(19) **Europäisches Patentamt / European Patent Office / Office européen des brevets**

(11) **EP 1 532 278 B1**

(12) **EUROPEAN PATENT SPECIFICATION**

(45) Date of publication and mention of the grant of the patent:
**22.02.2012 Bulletin 2012/08**

(51) Int Cl.:
**C12Q 1/68** (2006.01)

(21) Application number: **03792410.7**

(22) Date of filing: **21.08.2003**

(86) International application number:
**PCT/EP2003/009292**

(87) International publication number:
**WO 2004/018710 (04.03.2004 Gazette 2004/10)**

(54) **DIAGNOSIS OF CHRONIC REJECTION**

DIAGNOSE DER CHRONISCHEN ABSTOSSUNG

DIAGNOSTIC DE REJET CHRONIQUE

(84) Designated Contracting States:
**AT BE BG CH CY CZ DE DK EE ES FI FR GB GR HU IE IT LI LU MC NL PT RO SE SI SK TR**

(30) Priority: **22.08.2002 US 405225 P**

(43) Date of publication of application:
**25.05.2005 Bulletin 2005/21**

(73) Proprietors:
• **Novartis AG**
**4056 Basel (CH)**
Designated Contracting States:
**BE BG CH CY CZ DE DK EE ES FI FR GB GR HU IE IT LI LU MC NL PT**
• **Novartis Pharma GmbH**
**1230 Wien (AT)**
Designated Contracting States:
**AT**

(72) Inventors:
• **KRAUSE, Andreas**
**4123 Allschwil (CH)**
• **NIESE, Detlef**
**79100 Freiburg (DE)**
• **RAULF, Friedrich**
**79104 Freiburg (DE)**
• **SCHERER, Andreas**
**79618 Rheinfelden-Herten (DE)**

(74) Representative: **Graff, Alan**
**Novartis AG**
**Corporate Intellectual Property**
**4002 Basel (CH)**

(56) References cited:
• **SUTHANTHIRAN M: "Human renal allograft rejection: molecular characterization." NEPHROLOGY, DIALYSIS, TRANSPLANTATION: OFFICIAL PUBLICATION OF THE EUROPEAN DIALYSIS AND TRANSPLANT ASSOCIATION - EUROPEAN RENAL ASSOCIATION. ENGLAND 1998, vol. 13 Suppl 1, 1998, pages 21-24, XP002267088 ISSN: 0931-0509**
• **SHARMA V K ET AL: "Molecular correlates of human renal allograft rejection" TRANSPLANTATION PROCEEDINGS, vol. 30, no. 5, August 1998 (1998-08), pages 2364-2366, XP002267089 Meeting on New Dimensions in Transplantation: Weaving the Future;Florence, Italy; February 16-19, 1998 ISSN: 0041-1345**
• **TANG W H ET AL: "Activation of the serine proteinase system in chronic kidney rejection." TRANSPLANTATION. UNITED STATES 27 JUN 1998, vol. 65, no. 12, 27 June 1998 (1998-06-27), pages 1628-1634, XP008026499 ISSN: 0041-1337**
• **STREHLAU JURGEN ET AL: "Quantitative detection of immune activation transcripts as a diagnostic tool in kidney transplantation" PROCEEDINGS OF THE NATIONAL ACADEMY OF SCIENCES OF THE UNITED STATES, vol. 94, no. 2, 1997, pages 695-700, XP002267122 1997 ISSN: 0027-8424**
• **SUTHANTHIRAN MANIKKAM: "Clinical application of molecular biology: A study of allograft rejection with polymerase chain reaction" AMERICAN JOURNAL OF THE MEDICAL SCIENCES, vol. 313, no. 5, 1997, pages 264-267, XP008026444 ISSN: 0002-9629**

- **DELARUE FRANCOISE ET AL: "Prognostic value of plasminogen activator inhibitor type 1 mRNA in microdissected glomeruli from transplanted kidneys" TRANSPLANTATION (BALTIMORE), vol. 72, no. 7, 15 October 2001 (2001-10-15), pages 1256-1261, XP008026393 ISSN: 0041-1337**

- **KAMOUN MALEK: "Cellular and molecular parameters in human renal allograft rejection" CLINICAL BIOCHEMISTRY, vol. 34, no. 1, February 2001 (2001-02), pages 29-34, XP001176799 ISSN: 0009-9120**

**Description**

This invention relates to a method of monitoring the status of a transplanted tissue or organ in a recipient. In particular, the invention relates to the use of gene expression analysis to determine early prediction of chronic allograft rejection.

Chronic allograft rejection (CR) is the major cause for the failure of long-term graft survival. In contrast to treatable acute rejection episodes, chronic rejection is not reversible to date by any treatment when histologically detected, is not proven to be preventable by any immunosuppressive regimen and its pathogenesis is not fully understood but involving immunological as well as non-immunological factors. Characteristic for chronic rejection in all solid organ grafts is a concentric arterial intimal thickening by vascular remodeling. Kidney allografts with chronic rejection exhibit in addition pronounced parenchymal fibrosis and glomerular sclerosis: clinically, CR is manifested by a progressive decline in renal function, accompanied by proteinuria and hypertension.

Attempts to identify biomarkers in transplantation research have mainly been hypothesis-driven and lead to the characterization of individual genes, polymorphisms, biochemical or histopathological features. However, most of these studies were performed on tissue samples that already showed overt signs of disease and thus only revealed an association with the future severity of the disease.

There is a need to have a reliable tool for early prognosis and follow-up of CR, particularly early prognosis of CR before any overt clinical or histological manifestation, e.g. within the first year post transplantation; such an aid would be valuable e.g. for the optimisation of current treatment regimens and the design of clinical trials, including with new CR inhibiting agents.

The present invention relates to the identification of genes which are differentially expressed in transplant biopsies, e.g. renal biopsies, prior to the onset of CR in patients who will develop CR after the biopsy was taken, and patients that will not. The resulting gene expression pattern of a subset of the genes allows a highly statistically significant predictability of the occurrence of CR. For example, the genes identified in renal biopsies post transplantation before CR became histologically manifest, are indicated in Tables 1 (upregulated genes) and 2 (downregulated genes) and a subset of preferred genes in Table 3. The complete sequences of these 65 genes disclosed in this application are available using the GenBank accession number shown in Tables 1 to 3.

The genes identified according to the invention are useful predictive biomakers for the prognosis of CR in transplanted subjects. Any selection, of at least one, of these genes can be utilized as surrogate biomarker for early prognosis of CR. In particularly useful embodiments, a plurality of these genes, e.g. the 10 genes of Table 3 in case of renal biopsies, can be selected and their mRNA expression monitored simultaneoulsy to provide expression profiles for use in various aspects.

The invention provides for methods and uses as defined in the claims.

Accordingly, the invention provides the use of the KRT15 gene and the genesas listed in Table 3 as an early biomarker for chronic transplant rejection, e.g. as a biomarker for CR before any overt clinical or histological manifestation, e.g. within the first year post transplantation.

The methods of the present invention are to be performed *in vitro,* e.g. the levels of biomarkers are to be analysed in tissues or fluids extracted or obtained from a transplanted subject.

Methods of detecting the level of expression of mRNA are well-known in the art and include, but are not limited to, reverse transcription PCR, real time quantitative PCR, Northern blotting and other hybridization methods.

A particularly useful method for detecting the level of mRNA transcripts obtained from a plurality of the disclosed genes involves hybridization of labeled mRNA to an ordered array of oligonucleotides. Such a method allows the level of transcription of a plurality of these genes to be determined simultaneously to generate gene expression profiles or patterns. The gene expression profile derived from the biopsy obtained from the transplanted subject at risk of developing CR can be compared with the gene expression profile derived from the sample obtained from a transplanted subject that will not develop CR.

In a further embodiment, measuring expression profiles of one or a plurality of these genes or encoded proteins could provide valuable molecular tools for examining the efficacy of drugs for inhibiting, e.g. preventing or treating, CR. Changes in the expression profile from a baseline profile while the transplanted patient is exposed to therapy. Accordingly, this invention also provides a method for screening a transplanted subject to determine the likelihood that the subject will respond to the CR therapy, methods for the identification of agents that are useful in treating a transplanted subject having CR signs and methods for monitoring the efficacy of certain drug treatments for CR.

In one aspect, the invention features an *in vitro* method of identifying at least one gene which is differentially expressed in an allograft of a given tissue type prior to the onset of CR in a test transplanted subject compared to

i) a gene expression baseline profile originating from the same tissue type of a control transplanted subject who is known not to develop CR; or
ii) a gene expression baseline profile originating from the test transplanted subject at the date of the transplantation.

The term "differentially expressed" refers to a given allograft gene expression level and is defined as an amount which is substantially greater or less than the amount of the corresponding baseline expression level.

Accordingly, the method of diagnosing chronic rejection (CR) in a transplanted subject *in vitro* comprises

a) detecting as the baseline value the level of mRNA expression corresponding to the gene KRT15, said gene originating from a specific allograft tissue biopsy of a transplanted control subject who is known not to develop CR;

b) detecting a level of mRNA expression corresponding to the gene identified in a) in a renal allograft tissue biopsy obtained from a kidney transplanted test subject within 4 to 7 months post-transplantation; and

c) comparing the baseline value with the level of mRNA expression detected in step b) wherein the baseline value lower than the test value in the case of the genes KRT15 predicts that the kidney transplanted subject has an increased risk of developing CR.

Furthermore, the invention provides a method according to claim 1, further comprising:

a) detecting as the baseline value the level of mRNA expression corresponding to the gene hoxB7, NPRL2, OS9, G-protein $\gamma$7, OBMCL, DFKZp558B1722, EST ID 32f4, hoxA7 and PRLR, said genes originating from a specific allograft tissue biopsy of a transplanted control subject who is known not to develop CR;

b) detecting a level of mRNA expression corresponding to the genes identified in a) in a renal allograft tissue biopsy obtained from a kidney transplanted test subject within 4 to 7 months post-transplantation; and

c) comparing the baseline value with the level of mRNA expression detected in step b) wherein the baseline value lower than the test value in the case of the genes hoxB7, NPRL2, OS9, G-protein $\gamma$7, OBMCL, DFKZp558B1722, and EST ID 32f4 and higher than the test value, in the case of genes hoxA7 and PRLR, predicts that the kidney transplanted subject has an increased risk of developing CR.

In steps b) above, the level of mRNA or protein encoded is preferably detected within 4 to 7 months post-transplantation, more preferably around 6 months post-transplantation.

By prior to the onset of CR or early diagnosis of CR is meant before any overt clinical or histological manifestation of CR is detected in the transplanted subject.

The method of diagnosing CR according to the invention may also be applied to maintenance patients, i.e. patients who have been transplanted more than one year ago. Accordingly, biopsies are performed and the level of mRNA expression corresponding to at least one gene is compared to the level in the reference control values to identify patients that will develop CR during the next couple of months.

In another aspect, the invention provides a method for monitoring, e.g. preventing or inhibiting or reducing or treating CR in a transplanted subject at risk of developing CR with a CR inhibitor (e.g. a small molecule, an antibody or other therapeutic agent or candidate agent). Monitoring the influence of agents (e.g. drug compounds) on the level of expression of a marker of the invention can be applied not only in basic drug screening, but also in clinical trials. For example, the effectiveness of an agent to affect marker expression can be monitored in clinical trials of transplanted subjects receiving treatment for the inhibition of CR.

Such a method comprises:

a) obtaining a sample from a kidney transplanted subject prior to administration of a CR inhibiting agent,

b) detecting the level of expression of mRNA corresponding to the gene KRT15 in the sample obtained in step a) in a renal allograft tissue biopsy from said kidney transplanted subject ,

c) obtaining at least one sample from the kidney transplanted patient after administration of a CR inhibiting agent,

d) detecting the level of expression of mRNA corresponding to the gene KRT15 in the sample obtained in step c) in a renal allograft tissue biopsy from said kidney transplanted subject ,

e) comparing the level of expression of mRNA or protein detected in step b) wherein the baseline value lower than the test value in the case of the genes KRT15 predicts that the kidney transplanted subject has an increased risk of developing CR.

The invention also provides a method according to claim 4, further comprising:

a) detecting as the baseline value the level of mRNA expression corresponding to the gene hoxB7, NPRL2, OS9, G-protein $\gamma$7, OBMCL, DFKZp558B1722, EST ID 32f4, hoxA7 and PRLR, said genes originating from a specific allograft tissue biopsy of a transplanted control subject who is known not to develop CR;

b) detecting a level of mRNA expression corresponding to the genes identified in a) in a renal allograft tissue biopsy obtained from a kidney transplanted test subject within 4 to 7 months post-transplantation; and

c) comparing the baseline value with the level of mRNA expression detected in step b) wherein the baseline value lower than the test value in the case of the genes hoxB7, NPRL2, OS9, G-protein γ7, OBMCL, DFKZp558B1722, and EST ID 32f4 and higher than the test value, in the case of genes hoxA7 and PRLR, predicts that the kidney transplanted subject has an increased risk of developing CR.

Accordingly, incorporation of gene expression profiling data from human biopsies, e.g. human renal protocol biopsies, will help improve the patient selection process during clinical trials aimed at both treatment and prevention of the progression towards CR.

By transplanted subject is meant a subject receiving tissue or organ from a donor, preferably from the same species, e.g. kidney, heart, lung, combined heart and lung, liver, pancreas, bowel (e.g., colon, small intestine, duodenum), neuronal tissue, limbs.

Preferably more than one gene, e.g. a set of genes, are used in the methods of the invention. The methods of the invention are particularly preferred in kidney transplantation.

As already mentioned any selection, of at least one, of the genes indicated in Table 3 can be used.. Preferred genes of Table 3 are e.g.OS-9, NPRL2, HOXB7, G-CSF and/or BFGF. Most of the genes described here have not been implicated in renal allograft rejection and are associated with various functions. For example, OS9, also termed APRIL (acidic protein rich in leucines), is a member of the acidic nuclear phosphoprotein 32 family, with sequence homology to PHAPI, a putative HLA class II associated protein. OS9 also shares sequence similarity to the proto-oncogenes DEK and SET proteins, linked to myeloid leukemia. OBCML (opiate-binding protein/cell adhesion molecule-like) has only been described to be expressed in certain regions of the cerebellum, never in kidney. The tumor suppressor gene NPRL2, which shows sequence similarity to the yeast nitrogen permease regulator gene, has been shown to be located in the human chromosomal region 3p21.3, as one of 25 tumor suppressor genes within this region that are involved in the development of lung and breast cancer.

Gene expression profiles can be generated using e.g. the Affymetrix microarray technology. Microarrays are known in the art and consist of a surface to which probes that correspond in sequence to gene products (e.g. mRNAs, polypeptides, fragments thereof etc.). can be specifically hybridized or bound to a known position. Hybridization intensity data detected by the scanner are automatically acquired and processed by the GENECHIP$^R$ software. Raw data is normalized to expression levels using a target intensity of 200.

The transcriptional state of a cell may be measured by other gene expression technologies known in the art. Several such technologies produce pools of restriction fragments of limited complexity for electrophoretic analysis, such as methods combining double restriction enzyme digestion with phasing primers (e.g. EP-A1-0 534858), or methods selecting restriction fragments with sites closest to a defined mRNA end (e.g. Prashar et al, Proc. Nat. Acad. Sci., 93, 659-663, 1996). Other methods statistically sample cDNA pools, such as by sequencing sufficient bases (e.g. 20-50 bases) in each multiple cDNAs to identify each cDNA, or by sequencing short tags (e.g. 9-10 bases) which are generated at known positions relative to a defined mRNA end (e.g. Velculescu, Science, 270, 484-487,1995) pathway pattern.

In a preferred embodiment, the computation steps of the previous methods are implemented on a computer system or on one or more networked computer systems in order to provide a powerful and convenient facility for forming and testing models of biological systems. The computer system may be a single hardware platform comprising internal components and being linked to external components. The internal components of this computer system include processor element interconnected with main memory. The external components include mass data storage. This mass storage can be one or more hard disks. Other external components include user interface device, which can be a monitor and keyboards, together with pointing device or other graphic input devices. Typically, the computer system is also linked to other local computer systems, remote computer systems or wide area communication networks, e.g. Internet. This network link allows the computer system to share data and processing tasks with other computer systems.

Loaded into memory during operation of this system are several software components which are both standard in the art and special to the instant invention. These software components collectively cause the computer system to function according to the methods of this invention. These software components are typically stored on mass storage or on removable media, e.g. floppy disks or CD-ROM. The software component represents the operating system, which is responsible for managing the computer system and its network interconnections. preferably the methods of this invention are programmed in mathematical software packages, which allow symbolic entry of equations and high-level specification of processing, including algorithms to be used, and thereby freeing a user of the need to procedurally program individual equations or algorithms.

In preferred embodiments, the analytic software component actually comprises separate software components that interact with each other. Analytic software represents a database containing all data necessary for the operation of the system. Such data will generally include, but is not limited to, results of prior experiments, genome data, experimental procedures and cost, and other information, which will be apparent to those skilled in the art. Analytic software includes a data reduction and computation component comprising one or more programs which execute the analytic methods of the invention. Analytic software also includes a user interface which provides a user of the computer system with control and input of test network models and, optionally, experimental data. The user interface may comprise a drag-and-drop interface for specifying hypotheses to the system. The user interface may also comprise means for loading experimental data from the mass storage component, from removable media or from a different computer system communicating with the instant system over a network.

The invention also provides a process for preparing a database comprising at least one of the markers set forth in this invention, e.g. mRNAs. For example, the polynucleotide sequences are stored in a digital storage medium such that a data processing system for standardized representation of the genes that identify early prognosis of CR. The data processing system is useful to analyze gene expression between two tissue samples taken at different time point, e.g. at the transplantation day and post- transplantation. The isolated polynucleotides are sequenced. The sequences from the samples may be compared with the sequence(s) present in the database using homology search techniques. Alternative computer systems and methods for implementing the analytic methods of this invention will be apparent to one skilled in the art and are intended to be comprehended within the accompanying claims.

### Identification of Prognostic Markers of CR

As a part of a randomized, multicenter, double-blind, double-dummy, parallel group study, serial renal protocol biopsies are taken at the time of transplantation (baseline), then 6 months and 12 months after transplantation.

After RNA extraction from these biopsies, the overall yield of total RNA ranges from only around 10 ng to around 1,5 $\mu$g. Most of the samples contain less than 30 ng total RNA, which is far below the minimal amount of 1 to 5 $\mu$g that commercially available RNA labeling kits and methods need for subsequent microarray experiments. In order to obtain sufficient amounts of RNA for microarray experiments, linear RNA amplification is performed. Briefly, this method involves subsequent rounds of cDNA and aRNA synthesis, which amplifies the original amount 20-30 fold per round. 90 RNA samples are amplified and labeled. aRNAs are hybridized to Affymetrix HG U95A v2 chip containing oligonucleotide probes of about 12,000 human genes and analyzed.

### Tissue homogenization

All liquid nitrogen flash-frozen biopsy samples are stored in cryotubes at -80°C. Immediately after the addition of 700$\mu$l homogenization buffer (ABI lysis buffer/PBS 1:1) the homogenization step is performed by dipping the rod of a Polytron rotor/stator homogenizer PT 3100 into the tissue containing buffer and running the homogenizer at full speed for 30 seconds. If after this time remnant tissue pieces are visible, the procedure is repeated until homogenicity is achieved. Hereafter the homogenate is stored at -80°C until it is used in the RNA extraction step.

### Homogenate pre-filtration and RNA extraction

Pre-filtration of the homogenate and RNA extractions are performed by the ABI 6700 Biorobot workstation (Applied Biosystems, USA). Tissue homogenates are filled into the wells of a 96-deep-well plate, and placed in the filtrate position of the 6700 workstation. A tissue pre-filter tray is placed into the purification carriage and locked into position. The instrument door is closed, and the workstation software is launched.

The RNA extraction procedure includes a sample transfer step, a filtration step, a washing step, and an elution step. The sample transfer step, in which the pre-filtered homogenate is transferred from the 96 deep-well plate to the RNA purification tray includes a primary transfer of 550 $\mu$l solution. Before the second transfer, 150 $\mu$l homogenization buffer (Applied Biosystems lysis buffer/PBS 1:1) is added to each well in the deep-well plate, mixed three times and then 150 $\mu$l are transferred from there to the purification tray. The filtration step is carried out by applying a vacuum pressure of 80% for 180 seconds. The washing steps are performed as follows:

Step 1: washing solution 1, 400 $\mu$l, vacuum pressure 80% for 180 seconds, two times;
Step 2: washing solution 2, 500 $\mu$l, vacuum pressure 80% for 180 seconds, once;
Step 3: washing solution 2, 300 $\mu$l, vacuum pressure 60% for 120 seconds, two times.

A pre-elution vacuum of 90% pressure is applied for 300 seconds. Hereafter the elution step is performed by the addition of 120 $\mu$l elution solution (Applied Biosystems), and the application of a 100% vacuum-pressure for 120 seconds. The RNA samples are collected in 96-well plates (Applied Biosystems). The eluates are split into two aliquots of equal volume.

One aliquot is stored at - 80°C, the other aliquot is used for RNA amplification and GeneChip analysis.

### RNA amplification

Prior to the RNA amplification procedure, all RNA eluates are treated with RNeasy kit chemistry (Qiagen) to further clean the RNA from remnant salt or other substances that may inhibit the amplification efficiency. The volume of the aliquot is adjusted to 100 $\mu$l with RNase-free water. 350 $\mu$l buffer RLT is added and mixed thoroughly. 250 $\mu$l ethanol (96-100%) is added, and mixed thoroughly. The sample (700 $\mu$l) is applied to an RNeasy mini column, placed in a 2 ml collection tube. After a 15 second centrifugation step at more than 10,000 rpm, the flow-through is discarded. The RNeasy column is transferred into a new 2 ml collection tube. 500 $\mu$l buffer RPE is pipetted onto the column, the tube closed and centrifuged for 15 seconds at more than 10,000 rpm to wash the column. The flow-through is discarded. Another 500 $\mu$l buffer RPE is added to the column and the tube is centrifuged for 2 minutes at more than 10,000 rpm to dry the silica-gel membrane. To elute, the RNeasy column is transferred to a new 1,5 ml collection tube and 30 $\mu$l RNase-free water is added directly onto the membrane. After 1 minute incubation, the tube is centrifuged for 1 minute at more than 10,000 rpm to elute. This elution step is repeated once to get a total elution volume of 60 $\mu$l. The RNA is quantified by the Ribogreen method (Molecular Probes, Inc, USA). About 10 ng total RNA of each sample is used in three rounds of RNA amplification.

All enzymes and buffers for the amplification procedure are purchased from Invitrogen, Inc. (Carlsbad, CA, USA) unless explicitly mentioned. 10ml total RNA are incubated with 10 pmol T7-polydT primer [5'-GGCCAGTGAATT-GTAATACGACTCACTATAGGGAGGCGG(T)$_{24}$] (Genset, Inc.) in a volume of 11$\mu$l at 70°C for 10 minutes, then at 42°C for 5 minutes. The first strand reaction is carried out in a volume of 20$\mu$l by the addition of 200 units SuperScript II in the presence of first strand buffer, 10 mM DTT, 0.5 mM dNTP mixture, and 1$\mu$l RNase inhibitor (Ambion, Inc.) with a 42°C-incubation for 1 hr. The second strand synthesis is performed in 150 $\mu$l with 40 units E.coli DNA polymerase I in 1x second strand buffer, 0.2 mM dNTPs, 10 units E.coli DNA ligase and 2 units RNaseH. After a 2-hour incubation at 16°C, the double-stranded DNA is blunt-ended by the addition of 8 units T4 DNA polymerase for 10 minutes at 16°C. The double-stranded DNA product is purified with a QIAquick PCR purification kit (Qiagen) and eluted in 50 $\mu$l elution buffer. For only one round of amplification the volume of the eluate is reduced to dryness under vacuum, resuspended in 22 $\mu$l nuclease-free water, and then used in the RNA labelling reaction as described below. For additional rounds of amplification, the eluate is reduced to dryness under vacuum, resuspended in 8 $\mu$l nuclease-free water, and subjected to an in-vitro transcription reaction with the Ambion MEGAscript kit, following the manufacturer's instructions for a 20 $\mu$l reaction volume. After a 3-hour incubation at 37°C the RNA is purified with the RNeasy kit system (Qiagen). The RNA is eluted in 30 $\mu$l RNase free water, reduced to dryness under vacuum and resuspended in 11 $\mu$l nuclease-free water.

The second round of RNA amplification started with the addition of 1$\mu$l 0.1 mg/ml random hexamer primers followed by a 10-minute incubation at 70°C. The reaction mixture is chilled on ice and then incubated at room temperature for 10 minutes, at which point the first strand synthesis reaction is started by the addition of 200 units SuperScript II, 20 units RNase Inhibitor, 0.5 mM dNTPs and 10mM DTT in the presence of first strand reaction buffer. The mixture is incubated at 37°C for 1hour. A 20-minute RNase H treatment (2 units) at 37°C lead to the degradation of the residual RNA. RNase H is heat-inactivated at 94°C for 2 minutes and the mixture is chilled on ice. The second-strand synthesis is initiated by the addition of 100 pmol T7-polydT primer (see above) and incubation at 70°C for 5 minutes, followed by 42°C for 10 minutes. The second-strand synthesis is performed as described above, and the cDNA is purified with the QIAquick PCR purification kit (Qiagen). If this is the final round of amplification, the volume of the eluate is reduced to dryness under vacuum and resuspended in 22 $\mu$l nuclease-free water, followed by an in-vitro RNA labelling procedure (see below).

A third round of amplification is prepared by reducing the eluate to dryness under vacuum and resuspended in 8 $\mu$l nuclease-free water before it is then subjected to the procedure identical to that of a second round of amplification. We observed a 15 to 30-fold increase in aRNA in each round of amplification, resulting in 5x10$^5$ to 1.9x10$^6$-fold amplification of messenger RNA after three rounds (assuming 3.3 % poly(A)+ RNA within the initial pool of total RNA). Labelled RNAs are fractionated at 94°C for 35, 25, or 20 minutes for single (1 x), double (2x), or triple (3x) amplified RNAs, respectively. Shorter incubation times for 2x RNA and 3x RNA are chosen to avoid complete degradation of the RNA.

The RNA biotinylation step involved the use of the High-Yield RNA Labelling Kit (Enzo Diagnostics, NY, USA; P/N 900182) following the manufacturer's instructions. The following ingredients are mixed in an initial step:

> 22 $\mu$l aRNA
> 4 $\mu$l 10X HY reaction buffer,
> 4 $\mu$l 10X Biotin Labelled Ribonucleotides,
> 4 $\mu$l 10X DTT,
> 4 $\mu$l RNase inhibitor mix,
> 2 $\mu$l 20X T7 RNA polymerase.

The mixture is incubated at 37°C for 3-4 hours. The labelled aRNA is purified using RNeasy chemistry (Qiagen) following the manufacturer's instructions. The elution volume is 60 $\mu$l, 2 $\mu$l are used to determine the RNA concentration spectrophotometically by absorbance at 260 nm.

RNA fragmentation

15 μg labelled aRNA is fragmented in a volume of 20 μl by the addition of 4 μl 5X MES Fragmentation buffer and RNase free water. The mixture is incubated for 20 minutes at 94°C.

12X MES Fragmentation buffer (for 1000ml):

| | |
|---|---|
| 70,4 g | MES free acid |
| 193,3 g | (1,22M MES, 0.89M [Na$^+$] (2-(N-Morpholino)ethanesulfonic acid (SIGMA, P/N M5287) MES sodium salt (Sigma, P/N M3885) |
| 800 ml | DEPC water |

Filter through a 0.2 μm filter, the pH should be between 6.5 and 6.7 without adjustment.

Microarray hybridization mix

The hybridization is carried out in a volume of 300 μl. Fragmented aRNA is mixed with 150 μl 2X MES hybridization buffer, 3 μl herring sperm DNA (10mg/ml), 3 μl BSA (50mg/ml), 3 μl 948b control oligonucleotide (5nM), and 3 μl 20X Eukaryotic Hybridization Controls (Affymetrix). DEPC water is added to 300 μl final volume.

2X MES Hybridization buffer (for 500 ml):

| | |
|---|---|
| 217 ml | DEPC water |
| 200 ml | 5M NaCl |
| 82 ml | 12X MES |
| Filter through | 0.2 μm filter. |

Then add: 1.0 ml 10% Triton X-100. Store at room temperature.

Microarray pre-treatment

The microarray is incubated at 45°C for 15 minutes. The array chamber is filled with freshly prepared pre-treatment solution, prewarmed to 45°C.

Pre-treatment solution (300μl per microarray)

| | |
|---|---|
| 294 μl | 1X MES hybridization buffer |
| 3 μl | Acetylated BSA (50mg/ml) (Gibco BRL Life Technologies, P/N 15561-020) |
| 3 μl | Herring sperm DNA (10mg/ml) (Promega/Fisher scientific, P/N D1811) |

Microarray hybridization

While the microarrays are being pre-treated at 45°C, the hybridization mix is incubated at 99°C for 5 minutes. After a centrifugation for 5 minutes at 14,000 rpm the supernatant is transferred to a new Eppendorf tube and incubated at 45°C for 5 minutes. The pre-treatment solution is removed from the microarray chamber and replaced with the hybridization mix, avoiding bubbles. The septa of the plastic cartridge are covered with tape and the cartridge is placed in an oven at 45°C with the glass front facing down. The hybridization is continued for 16 to 18 hours.

Washing Procedure

The hybridization mix is removed from the probe array and set aside in a microcentrifuge tube. 280 μl 1X MES hybridization buffer is added to the chamber and a fluidics wash is performed on a GeneChip Fluidics Station 400 using 6X SSPE-T buffer.

6X SSPE-T wash buffer (1000ml)

300 ml 20X SSPE (BioWhittaker, P/N 16-010Y)
699 ml water

Filter through 0.2 μm filter. Add 1 ml 10% Triton X-100
After the fluidics wash the SSPE-T buffer is removed from the chamber and filled with stringent wash buffer, avoiding bubbles.
Stringent wash buffer (1000ml):

83.3 ml 12X MES buffer
5.2 ml 5M NaCl
1 ml 10% Tween 20
910.5 ml water

Filter through 0.2 μm filter. Add 1 ml 10% Triton X-100.
The microarray cartridges are layed face up in a 45°C incubation oven for 30 minutes. The stringent buffer is removed and the array is rinsed with 200 μl 1X MES hybridization buffer. The 1X MES hybridization buffer is completely removed, the array chamber filled with SAPE stain, and incubated at 37°C for 15 minutes.

SAPE stain (600 μl):

| 300 μl | 2x MES hybridization buffer |
|---|---|
| 288 μl | water |
| 6 μl | BAS (50mg/ml) |
| 6 μl | SAPE (1mg/ml) (Molecular probes, P/N 15230-147) |

After 15 minutes the SAPE stain solution is removed, the chamber filled with 200 μl 1X MES hybridization buffer, and a fluidics wash is performed. The SSPE-T solution is removed from the microrrarray chamber and replaced with 300 μl AB stain.

AS stain (300 μl):

| 150 μl | 2X MES hybridization buffer |
|---|---|
| 146.25 | μl water |
| 3 μl | BSA (50 mg/ml) |
| 0.75 | μl biotinylated antibody (500μg/ml) (Vector laboratories, P/N BA-0500) |

The cartridge is incubated at 37°C for 30 minutes, the AB stain is replaced with 200 μl 1X MES hybridization buffer, and a fluidics wash is performed. After the wash step, the SSPE-T solution is removed, the chamber is filled with SAPE stain, and incubated at 37°C for 15 minutes. The SAPE stain is replaced with 200 μl 1X MES hybridization buffer and a fluidics wash is performed. The septa are covered with tape to prevent buffer leakage.
Microarrays are scanned on Affymetrix GeneArray® scanners. Raw data sets are normalized by scaling 75%-quantile of all probe sets of each chip to a target intensity of 200.

Separation Method

Statistical analysis is performed with S-Plus (Insightful, Inc., USA) and GeneSpring[R] (Silicon Genetics, USA). Average difference values of less than 10 are rounded to 10. Low expression levels (between 10 and 50) are kept to ensure not to lose any possible pattern. Standard parametric and non-parametric statistics (Student's t-test, Wilcoxon's rank sum test) are applied.

More particularly, an algorithm is developed to separate the data for a particular gene for the CR and control group, a distinction being made between the CR group which has lower expression values than the control group and the CR group which has higher expression values than the control group. In addition to having separate expression ranges, the separation gap between the groups is maximized. Thus the gap between a specified quantile (Q) of the 2 data sets (i.e the $1-\alpha$ quantile of the group with lower expression levels and $\alpha$ quantile of the group with higher expression levels with $0<\alpha<0.5$).

The statistic to measure how well each gene separates the two groups is given as

$$S = \max (q_{\alpha, cr} - q_{1-\alpha, control}, q_{\alpha, control} - q_{1-\alpha, cr}, 0)$$

where $q_{\alpha\ cr}$ denotes the $\alpha$ quantile of the CR group with $0 < a < 0.5$ (and therefore $1 - \alpha > 0.5$). Similarly, control or cr denotes the control group.

The 0 is added to the evaluation of S because both terms, $q_{\alpha,cr} - q_{1-\alpha, control}$, and $q_{\alpha, control} - q_{1-\alpha, cr}$, can be negative. In this case, measuring the difference does not make any sense, as the groups overlay too much. In other words, the $1-\alpha$ quantile of the one group must be below the $\alpha$ quantile of the other.

An algorithm with following settings is used:

- Threshold (for noise) set to T = 10.
- Threshold (for minimum expression) set to $T_2 = 50$.
- The minimum percentage of signals above threshold $T_2$ is set in *each* group to P = 40%.
- The minimum percentage of signals above threshold $T_2$ over all groups combined is set to P = 50% (such that a higher percentage in one group can to some extent compensate a lower percentage in the other group).
- The percentage of signals below $T_2$ is determined for each gene, individually for each group (CR, control).
- All genes that do not fulfill all minimum percentage thresholds are discarded.
- The statistic S is calculated for each gene.
- The genes with S > 0 are returned, sorted by S.

As a first pass filter all genes containing a too large proportion of low signals are excluded. A gene is included in the dataset if

- both groups, CR and control, contain at least 40 percent values above the threshold $T_2 = 50$ (at least 4 out of 8 and 4 out of 9 samples).

- the combined dataset contains at least 50 percent values above the threshold T = 10 (at least 9 out of 17 samples).

In a second pass, all expression values below 10 are set to 10 to reduce random variation.

(The Affymetrix algorithm used to derive the expression levels can generate negative values). Low levels (between 10 and 50) are preferred to ensure not to lose any pattern that might occur.

In GeneSpring™, each gene is normalized to itself by creating a synthetic positive control for that gene, which is the mean of all values of that gene in a dataset, and dividing all measurements for that gene by this positive control, assuming it is at least 0.01.

TagMan Primer Probe Design

TaqMan assays should be designed to the region of a gene that hybridizes to the corresponding probe set of the HG-U95Av2 microarray. This region is called target sequence. Using the Netaffx™ software (Affymetrix, Inc), the target sequence of a probe set is identified. The target sequence is then imported into the program Primer Express (Applied Biosystems), and the primer/probe selection is performed by the program with the following conditions:

Primer TM (melting temperature) should be between 58°C and 60°C. Optimally it should be 59°C, with a maximum TM difference of 2°C.

The primer GC (GTP,CTP) content should be between 20% at the minimum and 80% at the maximum, avoiding any 3' GC clamps.

The optimal primer length should be 20 residues, but can range form 9 to 40 residues.

The amplicon requirements should be that the minimum TM is 0°C, the maximum TM 92°C. The amplicon should have a minimal length of 50 residues, a maximal length of 150.

TaqMan probe criteria are that the probe TM must be at least 0°C greater than the PCR primer TM, and the probe should not begin with a G (GTP) residue. If the target sequence is too short to identify any TaqMan assay matching the above mentioned criteria, the sequence is aligned to the entire sequence of the gene (using standard software such as GCG, Wisconsin Package, Accelrys, San Diego, CA) and a longer stretch of DNA is selected, encompassing the target sequence. Sequences of the forward primer, reverse primer and the TaqMan probe for each gene are listed in Table 4.

To identify the genes that separate best the chronic rejection group from the control group standard techniques are applied, e.g. t and Wilcoxon statistics, and a newly derived measure to find well-separated groups (with a large separation gap in between) is included.

The measure (Q15/85 and Q20/80) extends the concept of separation to finding a good separation gap. It is based on measuring the distance between the $\alpha$-quantile of the CR group and the $(1-\alpha)$-quantile of the control group and vice versa. If the $(\alpha, 1-\alpha)$ ranges of both groups do not overlap, the maximum distance is reported, otherwise 0. Applying the measures to each gene individually delivers a measure for the separation of the 2 groups. The Q20/80 method identified 65 genes and the Q15/85 method 16 genes with complete separation of the (20%, 80%) and (15%, 85%) quantile ranges, respectively.

Comparing the genes detected to the ordered t- and Wilcoxon statistic identified that 10 of those genes are ranked among the 100 with most extreme t- and Wilcoxon statistic. The gene identifiers and annotations are given e.g. in table 3.

Almost all genes identified show expression levels above the low threshold of 50, mostly even much higher.

Applying a different way of evaluating the power of a set of genes to separate the two groups of samples, a cluster analysis of all 65 genes that are common to both, the t statistic list and the Wilcoxon list is performed. The cluster is prepared in two steps. First, a gene cluster is prepared by standard correlation, then an array cluster by Pearson correlation.

To make a tree, GeneSpring calculates the correlation for each gene with every other gene in the set. Then it takes the highest correlation and pairs those two genes, averaging their expression profiles. GeneSpring then compares this new composite gene with all of the other unpaired genes. This is repeated until all of the genes have been paired. At this point the minimum distance and the separation ratio come into play. Both of these affect the branching behavior of the tree. The minimum distance deals with how far down the tree discrete branches are depicted. The number specified in the minimum distance box determines the minimum separation considered significant between genes. This reduces meaningless structure at the base of the tree. Decreasing minimum distance increases the 'branchiness' of the tree. Default minimum distance is 0.001. A value smaller than 0.001 has very little effect, because most genes are not correlated more closely than that. A higher number will tend to lump together more genes into a group, making the groups less specific.

This number should be between 0 and 1. The separation ratio determines how large the correlation difference between groups of clustered genes has to be for the groups to be considered discrete groups and not be joined together. Increasing separation increases the 'branchiness' of the tree. The default separation ratio is 0.5, but it can range from 0.0 to 1.0. At a separation ratio of 0, all gene expression profiles can be regarded as identical.

The Pearson correlation is very similar to the Standard correlation, except it measures the angle of expression vectors for genes A and B around the mean of the expression vectors (for example, the mean of the expression values constituting the profiles for Gene A and Gene B). Generally the mean of the expression vectors will be positive since expression values are based on concentrations of mRNA. Using the Pearson correlation more negative correlations are obtained than from the Standard correlation. It is worth noting that the Pearson correlation gives you almost the same correlations as the Standard correlation when they are both performed on the logarithms of the genes' expression values. This is how to compute a Pearson correlation: calculate the mean of all elements in vector a. Then subtract that value from each element in a. Call the resulting vector A. Do the same for b to make a vector B. Pearson Correlation = A.B / ( I A II B I )

The gene cluster is prepared by performing a standard correlation analysis with a separation ratio value of 0.5 and a minimum distance value of 0.001. For the microarray cluster a Pearson correlation is used with a separation ratio of 1.0 and a minimum distance value of 0.001. Accordingly, the set of 65 genes separates the two patient groups perfectly. The distance between the two groups is rather small. This was expected since all patients were clinically and histologically healthy at the point these biopsies were drawn.

By applying above method the expression levels of about 12,000 transcripts in serial renal allograft protocol biopsies from 17 transplant patients have been monitored. Demographic and clinical characteristics of all patients in this study are listed in Table 5. One group of patients developed CR within 6 months after the timepoint the biopsy was taken, the other group did not. Using the set of genes as disclosed, preferably the set of 10 genes as indicated in Table 3, as identified by detailed statistical analysis of the biopsy RNA expression profiles, the occurrence/non occurrence of chronic rejection was predicted in 15 out of these 17 patients (> 88 %). Furthermore, the set of discriminator genes was also able to predict that a month 12 biopsy belonged to a patient that developed CR until month 18.

Table 1: List of genes (with GenBank accession numbers) which are upregulated in pre-CR group

| GenBank accession number | Affymetrix probe set | description | fold change | Wilcoxon p-value | t-test p-value |
|---|---|---|---|---|---|
| X07696 | 37582_at | cytokeratin 15 / KRT15 | 7.96 | 0.0003 | 0.0192 |
| AB005666 | 36843_at | GTPase-activating protein | 6.93 | 0.0064 | 0.0284 |
| U65785 | 33863_at | ORP150 mRNA | 6.54 | 0.0193 | 0.0051 |
| M16937 | 37618_at | homeobox c1 protein / hoxB7 | 5.73 | 0.0009 | 0.0197 |
| Z37166 | 35292_at | BAT1 mRNA (DEAD family) | 5.50 | 0.0266 | 0.0163 |
| U48231 | 33549_at | bradykinin B1 receptor | 4.88 | 0.0030 | 0.0108 |
| U77968 | 34652_at | member of the bHLH-PAS family | 4.55 | 0.0047 | 0.0059 |
| AJ000480 | 35597_at | C8FW phosphoprotein | 4.30 | 0.0046 | 0.0089 |
| AF040708 | 40499_r_at | NPRL2 / similar to yeast NPR2 nitrogen permease | 4.28 | 0.0041 | 0.0058 |
| AJ011497 | 38482_at | claudin-7 | 4.34 | 0.0193 | 0.0044 |
| Z70519 | 37644_s_at | FAS gene missing exon 4 | 4.41 | 0.0064 | 0.0103 |
| U41635 | 36996_at | OS-9 | 2.70 | 0.0011 | 0.0388 |
| U45448 | 33535_at | ATP-gated ion channel | 2.69 | 0.0104 | 0.0167 |
| AB010414 | 39893_at | G-protein gamma 7 | 2.54 | 0.0030 | 0.0186 |
| X98411 | 35132_at | myosin-IF | 2.47 | 0.0152 | 0.0240 |
| AB011082 | 36408_at | ORCTL4 | 2.38 | 0.0289 | 0.0230 |
| X63546 | 1613_s_at | tre oncogene | 3.05 | 0.0068 | 0.0048 |
| S80864 | 35955_at | putative protein | 3.01 | 0.0289 | 0.0189 |
| A1827895 | 36224_g_at | IMAGE:2350347 | 2.98 | 0.0152 | 0.0406 |
| U13897 | 40246_at discs | human homolog of Drosophila discs large protein | 2.88 | 0.0071 | 0.0072 |
| U16799 | 37669_s_at | Na,K-ATPase beta-1 subunit | 2.88 | 0.0211 | 0.0163 |
| X14445 | 1855_at | int-2 (FGF-3) | 2.92 | 0.0107 | 0.0406 |
| A1653621 | 36992_at | thioredoxin | 3.12 | 0.0101 | 0.0108 |

(continued)

| GenBank accession number | Affymetrix probe set | description | fold change | Wilcoxon p-value | t-test p-value |
|---|---|---|---|---|---|
| U79251 | 41093_at | OBCML, opioid binding protein/cell adhesion molecule-like | 3.48 | 0.0047 | 0.0166 |
| M59818 | 34223_at | granulocyte colony-stimulating factor receptor (G-CSFR-1) | 3.58 | 0.0095 | 0.0310 |
| D11086 | 1506_at | interleukin 2 receptor gamma chain | 3.71 | 0.0149 | 0.0146 |
| NM002893 | 1515_at | retinoblastoma binding protein 7 | 4.01 | 0.0106 | 0.0281 |
| AL049449 | 33997_at | DKFZp586B1722 | 4.08 | 0.0107 | 0.0333 |
| AL049228 | 31985_at | DKF7p564N1716 | 4.10 | 0.0249 | 0.0228 |
| X64116 | 32698_at | poliovirus receptor | 1.92 | 0.0107 | 0.0134 |
| S85655 | 36592_at | prohibitin | 1.94 | 0.0152 | 0.0098 |
| W26469 | 31377_r_at | EST ID 32f4 from retina cDNA randomly primed sublibrary | 2.23 | 0.0152 | 0.0208 |
| AB020638 | 33226_at | KIAA0876 | 2.25 | 0.0152 | 0.0402 |

Table 2: List of genes (with GenBank accession numbers) early downregulated in pre-CR group

| GenBank accession number | Affymetrix probe set | description | fold change | Wilcoxon p-value | t-test p-value |
|---|---|---|---|---|---|
| U56637 | 40910_at | capping protein alpha subunit isoform 1 | 4.16 | 0.0010 | 0.0036 |
| D14110 | 1276_g_at | RNA binding protein | 3.76 | 0.0030 | 0.0135 |
| M24194 | 34609_g_at | homologue; putative (G-protein) | 3.71 | 0.0046 | 0.0168 |
| X56777 | 39720_g_at | ZP3 | 3.48 | 0.0095 | 0.0301 |
| U94747 | 38171_at | WD repeat protein; similar to petunia AN11 | 3.49 | 0.0172 | 0.0282 |
| AB018313 | 39130_at | KIAA0770 | 3.47 | 0.0211 | 0.0469 |
| U66059 | 32795_at | T cell receptor beta locus | 3.30 | 0.0203 | 0.0410 |
| M93651 | 40189_at | SET gene | 5.97 | 0.0028 | 0.0141 |
| AJ005814 | 40343_at | hoxA7 | 5.03 | 0.0055 | 0.0549 |

(continued)

| GenBank accession number | Affymetrix probe set | description | fold change | Wilcoxon p-value | t-test p-value |
|---|---|---|---|---|---|
| X75861 | 33988_at | TEGT | 5.27 | 0.0071 | 0.0470 |
| X78947 | 36638_at | connective tissue growth factor | 5.22 | 0.0208 | 0.0489 |
| L05095 | 31708_at | ribosomal protein L30 | 10.83 | 0.0211 | 0.0579 |
| U52112 | 31873_at | ARD1 subunit homolog | 3.01 | 0.0046 | 0.0106 |
| AF031416 | 35960_at | IKK beta | 3.03 | 0.0149 | 0.0076 |
| M31661 | 1079_g_at | prolactin (PRL) receptor | 3.09 | 0.0180 | 0.0231 |
| Z25749 | 34646_at | ribosomal protein S7 | 2.95 | 0.0072 | 0.0185 |
| AF034176 | 32218_at | clone ntcon5 contiq | 2.87 | 0.0106 | 0.0285 |
| U90904 | 38452_at | clone 23773 | 2.87 | 0.0104 | 0.0430 |
| D38048 | 39060_at | proteasome subunit z | 2.80 | 0.0193 | 0.0356 |
| A1743134 | 41246_at | similar to glia derived nexin precursor | 2.81 | 0.0212 | 0.0383 |
| U66078 | 33972_r_at | DAZLA | 2.58 | 0.0048 | 0.0053 |
| AB023154 | 35369_at | KIAA0937 | 2.66 | 0.0072 | 0.0133 |
| AI540925 | 41206_r_at | PEC1.2_15_A02.r cDNA | 2.26 | 0.0072 | 0.0145 |
| U07132 | 519_g_at | Ner-I steroid hormone receptor | 2.29 | 0.0149 | 0.0267 |
| AA527880 | 35774_r_at | NDUFB7 | 2.38 | 0.0152 | 0.0356 |
| AI828210 | 38592_s_at | IMAGE:2421832 | 2.35 | 0.0212 | 0.0165 |
| U77327 | 32970_f_at | CD30 | 1.82 | 0.0107 | 0.0211 |
| U40282 | 35365_at | integrin-linked kinase | 1.80 | 0.0212 | 0.0154 |
| AF029778 | 32137_at | jagged 2 (Notch ligand) | 1.84 | 0.0289 | 0.0422 |
| M92287 | 1795_g_at | cyclin D3 (CCND3) | 1.89 | 0.0289 | 0.0193 |
| AF042379 | 39918_at | GCP2 | 2.00 | 0.0107 | 0.0205 |
| X56468 | 409_at | 14.3.3tau | 2.12 | 0.0152 | 0.0210 |

Table 3: Subset of 10 genes from tables 1 and 2 with the most significant differential expression patterns for the pre-CR and the control group. The expression pattern of eight genes are validated by TaqMan™ real-time Q-PCR. nd: not done due to limited sequence information.

| accession number | name | description | fold change (HG-U95) | fold change (Q-PCR) |
|---|---|---|---|---|
| **upregulated in preCR group at month 6** | | | | |
| X07696 | KRT15 | cytoskeletal structural protein | 7.96 | 2.36 |
| M16937 | hoxB7 | homeodomain family of DNA binding proteins | 5.73 | 2.55 |
| AF040708 | NPRL2 | candidate tumor suppressor gene 21 protein | 4.28 | 4.60 |
| U41635 | OS9 | acidic (leucine-rich) nuclear phosphoprotein | 2.70 | 4.20 |
| AB010414 | G-protein γ 7 | guanine nucleotide binding protein | 2.54 | 4.99 |
| U79251 | OBCML | immunoglobulin protein superfamily member | 3.48 | 20.89 |
| AL049449 | DKFZp5586B1722 | uncharacterized | 4.08 | 4.21 |
| W26469 | EST ID 32f4 | uncharacterized | 2.23 | nd |
| **downregulated in preCR group at month 6** | | | | |
| AJ005814 | hoxA7 | homeodomain transcription factor | -5.03 | 1.86 |
| M31661 | PRLR | prolactin receptor | -3.09 | -32.31 |

Table 4: Sequences and labels of all probes and primers used in TaqMan™ assays.

| GenBank accession # | Name | Sequence |
|---|---|---|
| X07696 | KRT15 | 5'-GGCTTTGCATGCGCTCTATT-3' <br> 5'-GCTGCATCTCCTTGCTCCA-3' <br> 5'-FAM-CCCCTCTGCCTCTCCCCACCTTC-TAMRA-3' |
| M16937 | HoxB7 | 5'-GGAGCCCCAAAAACCTACCA-3' <br> 5'-AAGCAAGAAGCAGCAGCCA-3' <br> 5'-FAM-TCGCGTGTTCCCCAAGCGC-TAMRA-5' |
| AF040708 | NPR2 | 5'-TGGGAGTTACCTGAGGGAAGC-3' <br> 5'-GATTGGCAGTGCCCCATG-3' <br> 5'-FAM-AGACCCTTTATGTCTCTCAGGAGCCCTGGA-TAMRA-3' |
| U41635 | OS9 | 5'-GCAAGGAGGGCAGGACACT-3' <br> 5'-CAAACATCACTAAGGGCAGGTG-3' <br> 5'-FAM-CAGGCACTGAGCAAGCAGGCCC-TAMRA-3' |
| AB010414 | G-protein γ 7 | 5'-TGGCCTTCTCAGTTTGGGC-3' <br> 5'-TTCAGTTATTCCGAACGGGAA-3' <br> 5'-FAM-AAAGGGATGGAGGCTTTACGGCCA-TAMRA-3' |
| U79251 | OBCML | 5'-CTGAGCCACCTTTGCTGTCTT-3' <br> 5'-TTTGAATCCCAGGCAACTTTG-3' <br> 5'-FAM-TCTCCTGGGACGAGAAGGACTCATCCA-TAMRA-3' |

(continued)

| GenBank accession # | Name | Sequence |
|---|---|---|
| AL049449 | DKFZp586B1722 | 5'-AACTTGCCAATTCTGTGAATGTTATT-3'<br>5'-GGGACATGTTACCCAATCACAA-3'<br>5'-FAM-ATTTAAAAAGCTGGGTCTGTAATGGGAGGCATT-TAMRA-3' |
| AJ005814 | HoxA7 | 5'-TGGAAATTCTGCTCACTTCTTGC-3'<br>5-TCTGATGTCATGGCCAAATTTG-3'<br>5'-FAM-CTTGCTTGCTTCTCTGGTGGGCTTCC-TAMRA-3' |
| M31661 | PRLR | 5'-GACACTACTAAAGCTCCCAGCTCC-3'<br>5'-TTCTGGAATCAGCTGCTGGA-3'<br>5'-FAM-TTCATGCTCCATTTTTAACCACTTGCCTCTT-TAMRA-3' |

Table 5: Demographics of the recipients and donors.

| | recipient | | | | | | | | donor | | | | | HLA |
|---|---|---|---|---|---|---|---|---|---|---|---|---|---|---|
| patient | age | gender | ethnicity | endstage renal disease | AR | month 12 histology diagnosis | BANFF grading | | age | gender | ethnicity | type of tx | | HLA Mismatch al A-8-DR loci |
| A | 41 | F | Ot | GN/GD | 1 | 1 | mild | | 22 | M | ot | CadHB | | 1-1-2 |
| B | 27 | M | Ca | GN/GD | 0 | 1 | mild | | 26 | F | Ca | LivUnrel | | 1-2-2 |
| C | 42 | M | Ca | DM | 0 | 1 | mild | | 39 | F | Ca | CadHB | | 1-1-0 |
| D | 62 | M | Ca | GN/GD | 1 | 1 | mild | | 53 | M | Ca | CadHB | | 1-1-1 |
| E | 54 | M | Ca | GN/GD | 0 | 1 | mild | | 44 | F | Ca | CadHB | | 2-1-2 |
| F | 50 | M | Ca | other | 0 | 1 | mild | | 55 | F | Ca | LivRet | | 1-1-1 |
| G | 27 | M | Ca | other | 0 | 1 | mild | | 55 | F | Ca | Liv rel | | 1-1-1 |
| H | 39 | M | Ca | Unkn | 0 | 1 | moderate | | 47 | F | Ca | LivUnrel | | 1-2-2 |
| I | 49 | F | Ca | PyN/IN | 0 | 1 | mild | | 47 | F | Ca | LivUnrel | | 2-2-1 |
| J | 19 | F | Bl | Unkn | 0 | 0 | none | | 42 | F | Bl | LivRel | | 1-1-0 |
| K | 50 | F | Ca | Unkn | 0 | 0 | none | | 23 | F | Ca | LivRel | | 0-0-0 |
| L | 53 | M | Ca | DM | 0 | 0 | none | | 62 | M | Ca | CadHB | | 1-0-1 |
| M | 47 | F | Ca | PCKD | 1 | 0 | none | | 51 | M | ot | CadHB | | 2-0-0 |
| N | 30 | F | Ot | GN/GD | 0 | 0 | none | | 59 | M | 01 | LivRel | | 0-1-1 |
| O | 37 | F | Ca | Htn/Nsc | 0 | 0 | none | | 42 | F | Ca | LivRel | | 1-1-1 |
| P | 30 | M | Bl | Unkn | 1 | 0 | none | | 27 | M | Bl | LivRel | | 2-2-2 |
| Q | 21 | F | ot | GN/GD | 0 | 0 | none | | 43 | F | ot | LivRel | | 0-0-0 |

Recipients A - I developed CR between month 6 and month 12, patients J - Q remained healthy. AR: number of acute rejection episodes;
Gender: F, female; M, male. Ethnicity: Ot, oriental; Ca, caucasian; Bl, black. End stage renal disease: GN/GD, glomerulonephritis/glomerular disease; PyN/IN, pyelonephritis/interstitial nephritis; PCKD, polycystic kidney disease; Htn/Nsc, hypertension/nephrosclerosis; Vsc, vasculitis; DM = diabetes mellitus; OD/R, obstructive disorder / reflux; Unk, unknown origin. AR: number of acute rejection episodes. Month 12 histology diagnosis: 1, Chronic rejection positive; 0, no rejection. Type of transplant: CadHB, cadaveric heart beating; CadNHB, cadaveric non-heart beating; LivRel, living related; LivUnrel, living unrelated.

**Claims**

1. A method of diagnosing chronic rejection (CR) in a transplanted subject *in vitro* comprising

   a) detecting as the baseline value the level of mRNA expression corresponding to the gene KRT15, said gene originating from a specific allograft tissue biopsy of a transplanted control subject who is known not to develop CR;
   b) detecting a level of mRNA expression corresponding to the gene identified in a) in a renal allograft tissue biopsy obtained from a kidney transplanted test subject within 4 to 7 months post-transplantation; and
   c) comparing the baseline value with the level of mRNA expression detected in step b) wherein the baseline value lower than the test value in the case of the genes KRT15 predicts that the kidney transplanted subject has an increased risk of developing CR.

2. A method according to claim 1, further comprising:

   a) detecting as the baseline value the level of mRNA expression corresponding to the gene hoxB7, NPRL2, OS9, G-protein $\gamma7$, OBMCL, DFKZp558B1722, EST ID 32f4, hoxA7 and PRLR, said genes originating from a specific allograft tissue biopsy of a transplanted control subject who is known not to develop CR;
   b) detecting a level of mRNA expression corresponding to the genes identified in a) in a renal allograft tissue biopsy obtained from a kidney transplanted test subject within 4 to 7 months post-transplantation; and
   c) comparing the baseline value with the level of mRNA expression detected in step b) wherein the baseline value lower than the test value in the case of the genes hoxB7, NPRL2, OS9, G-protein $\gamma7$, OBMCL, DFKZp558B1722, and EST ID 32f4 and higher than the test value, in the case of genes hoxA7 and PRLR, predicts that the kidney transplanted subject has an increased risk of developing CR.

3. A method according to claim 1 or 2, wherein the allograft tissue biopsy is obtained from the control subject at the day of transplantation.

4. A method according to claims 1 to 3, wherein in step b), the level of mRNA expression corresponding to the gene (s) selected in step a) in a renal allograft biopsy obtained from a kidney transplanted test subject is detected at around 6 months post-transplantation.

5. A method for monitoring CR in vitro in a transplanted subject at risk of developing CR comprising a) obtaining a pre-administration sample from a kidney transplanted subject prior to administration of a CR inhibiting agent, b) detecting the level of expression of mRNA corresponding to the gene KRT15 in the pre-administration sample, c) obtaining one or more post-administration samples from the kidney transplanted patient, d) detecting the level of expression of mRNA corresponding to the gene KRT15 in the post-administration kidney sample or samples, e) comparing the level of expression of mRNA corresponding to the gene KRT15 in the pre-administration sample with the level of expression of mRNA of said gene in the post-administration sample or samples, and f) adjusting the agent accordingly.

6. A method according to claim 5, further comprising:

   a) detecting the level of mRNA expression corresponding to the genes hoxB7, NPRL2, OS9, G-protein $\gamma7$, OBMCL, DFKZp558B1722, EST ID 32f4, hoxA7 and PRLR, said genes originating from a specific allograft tissue biopsy of a transplanted control subject who is known not to develop CR;
   b) detecting a level of mRNA expression corresponding to the genes identified in a) in a renal allograft tissue biopsy obtained from a kidney transplanted test subject within 4 to 7 months post-transplantation; and
   c) comparing the level of step a.) with the level of mRNA expression detected in step b) wherein the level of step a.) lower than the level of step b.) in the case of the genes hoxB7, NPRL2, OS9, G-protein $\gamma7$, OBMCL, DFKZp558B1722, and EST ID 32f4 and higher than the level of step b.) in the case of genes hoxA7 and PRLR, predicts that the kidney transplanted subject has an increased risk of developing CR.

7. A method according to any one of claims 1 to 6, wherein the level of mRNA expression of one or more genes is detected by techniques selected from the group consisting of Northern blot analysis, reverse transcription PCR and real time quantitative PCR.

8. Use of the gene KRT15 as a biomarker for chronic transplant rejection according to the method of claim 1.

9. The use according to claim 8, further comprising the genes hoxB7, NPRL2, OS9, G-protein $\gamma7$, OBMCL,

DFKZp558B1722, EST ID 32f4, hoxA7 and PRLR.

**10.** The use according to claim 8 or 9, as a biomarker for chronic transplant rejection within 4 to 7 months post-transplantation.

**11.** The use according to any one of claims 8 to 10, as a biomarker for chronic transplant rejection at around 6 months.

**Patentansprüche**

**1.** Verfahren zum in vitro-Diagnostizieren einer chronischen Abstoßung (CR) bei einem transplantierten Individuum, umfassend:

a) Nachweisen als Basislinienwert des Grads der dem Gen KRT15 entsprechenden mRNA-Expression, wobei das Gen aus einer speziellen Allotransplantat-Gewebebiopsie eines transplantierten Kontrollindividuums stammt, von dem bekannt ist, dass es keine CR entwickelt;
b) Nachweisen eines Grads einer dem in a) identifizierten Gen entsprechenden mRNA-Expression in einer Nierenallotransplantat-Gewebebiopsie, die von einem nierentransplantierten Testindividuum innerhalb von 4 bis 7 Monaten nach Transplantation erhalten wird; und
c) Vergleichen des Basislinienwerts mit dem Grad der in Stufe b) nachgewiesenen mRNA-Expression, wobei der Basislinienwert, der niedriger ist als der Testwert, im Falle der Gene KRT15 vorhersagt, dass das nierentransplantierte Individuum ein erhöhtes Risiko aufweist, eine CR zu entwickeln.

**2.** Verfahren nach Anspruch 1, des Weiteren umfassend:

a) Nachweisen als Basislinienwert des Grads der mRNA-Expression, die den Genen hoxB7, NPRL2, OS9, G-Protein $\gamma$7, OBMCL, DFKZp558B1722, EST ID 32f4, hoxA7 und PRLR entspricht, wobei die Gene aus einer speziellen Allotransplantat-Gewebebiopsie eines transplantierten Kontrollindividuums stammen, von dem bekannt ist, dass es keine CR entwickelt;
b) Nachweisen eines Grads einer den in a) identifizierten Genen entsprechenden mRNA-Expression in einer Nierenallotransplantat-Gewebebiopsie, die von einem nierentransplantierten Testindividuum innerhalb von 4 bis 7 Monaten nach Transplantation erhalten wird; und
c) Vergleichen des Basislinienwerts mit dem Grad der in Stufe b) nachgewiesenen mRNA-Expression, wobei der Basislinienwert, der im Falle der Gene hoxB7, NPRL2, OS9, G-Protein $\gamma$7, OBMCL, DFKZp558B1722 und EST ID 32f4 niedriger ist als der Testwert und im Falle der Gene hoxA7 und PRLR höher ist als der Testwert, vorhersagt, dass das nierentransplantierte Individuum ein erhöhtes Risiko aufweist, eine CR zu entwickeln.

**3.** Verfahren nach Anspruch 1 oder 2, wobei die Allotransplantat-Gewebebiopsie aus dem Kontrollindividuum am Tag der Transplantation erhalten wird.

**4.** Verfahren nach den Ansprüchen 1 bis 3, wobei in Stufe b) der Grad der mRNA-Expression, die dem (den) in Stufe a) ausgewählten Gen (Genen) entspricht, in einer Nierenallotransplantat-Biopsie, die von einem nierentransplantierten Testindividuum erhalten wird, etwa 6 Monate nach der Transplantation nachgewiesen wird.

**5.** Verfahren zum Überwachen einer CR in vitro bei einem transplantierten Individuum, das das Risiko aufweist, eine CR zu entwickeln, umfassend:

a) Gewinnen einer vor Verabreichung erhaltenen Probe von einem nierentransplantierten Individuum vor einer Verabreichung eines CR-hemmenden Mittels,
b) Nachweisen des Expressionsgrads der dem Gen KRT15 entsprechenden mRNA in der vor Verabreichung erhaltenen Probe,
c) Gewinnen einer oder mehrerer nach Verabreichung erhaltenen Probe(n) von dem nierentransplantierten Patienten,
d) Nachweisen des Expressionsgrads der dem Gen KRT15 entsprechenden mRNA in der (den) nach Verabreichung erhaltenen Nierenprobe oder Nierenproben,
e) Vergleichen des Expressionsgrads der dem Gen KRT15 entsprechenden mRNA in der vor Verabreichung erhaltenen Probe mit dem Expressionsgrad der mRNA dieses Gens in der (den) nach Verabreichung erhaltenen Probe oder Proben, und

f) entsprechendes Einstellen des Mittels.

6. Verfahren nach Anspruch 5, das des Weiteren umfasst:

a) Nachweisen des Grads der mRNA-Expression, die den Genen hoxB7, NPRL2, OS9, G-Protein γ7, OBMCL, DFKZp558B1722, EST ID 32f4, hoxA7 und PRLR entspricht, wobei diese Gene aus einer speziellen Allotransplantat-Gewebebiopsie eines transplantierten Kontrollindividuums stammen, von dem bekannt ist, dass es keine CR entwickelt;

b) Nachweisen eines Grads einer den in a) identifizierten Genen entsprechenden mRNA-Expression in einer Nierenallotransplantat-Gewebebiopsie, die von einem nierentransplantierten Testindividuum innerhalb von 4 bis 7 Monaten nach Transplantation erhalten wird; und

c) Vergleichen des Grads von Stufe a) mit dem Grad der in Stufe b) nachgewiesenen mRNA-Expression, wobei der Grad von Stufe a), der im Falle der Gene hoxB7, NPRL2, OS9, G-Protein γ7, OBMCL, DFKZp558B1722 und EST ID 32f4 niedriger ist als der Grad von Stufe b) und im Falle der Gene hoxA7 und PRLR höher ist als der Grad von Stufe b), vorhersagt, dass das nierentransplantierte Individuum ein erhöhtes Risiko aufweist, eine CR zu entwickeln.

7. Verfahren nach einem der Ansprüche 1 bis 6, wobei der Grad der mRNA-Expression eines Gens oder mehrerer Gene durch Techniken nachgewiesen wird, die aus der Gruppe ausgewählt sind, die aus Northern Blot-Analyse, Reverse Transkription-PCR und quantitativer Echtzeit-PCR ausgewählt sind.

8. Verwendung des Gens KRT15 als Biomarker für eine chronische Transplantatabstoßung gemäß dem Verfahren von Anspruch 1.

9. Verwendung nach Anspruch 8, wobei des Weiteren die Gene hoxB7, NPRL2, OS9, G-Protein γ7, OBMCL, DFKZp558B1722, EST ID 32f4, hoxA7 und PRLR umfasst sind.

10. Verwendung nach Anspruch 8 oder 9 als Biomarker für eine chronische Transplantatabstoßung innerhalb von 4 bis 7 Monaten nach Transplantation.

11. Verwendung nach einem der Ansprüche 8 bis 10 als Biomarker für eine chronische Transplantatabstoßung nach etwa 6 Monaten.

**Revendications**

1. Procédé de diagnostic *in vitro* du rejet chronique (RC) chez un sujet transplanté, comprenant :

a) la détection en tant que valeur en ligne de base du niveau d'expression d'ARNm correspondant au gène KRT15, ledit gène provenant d'une biopsie de tissu d'allogreffe spécifique d'un sujet de contrôle transplanté qui est connu pour ne pas développer de RC ;

b) la détection d'un niveau d'expression d'ARNm correspondant au gène identifié en a) dans une biopsie de tissu d'allogreffe de rein obtenue chez un sujet test ayant subi une transplantation rénale dans les 4-7 mois suivant la transplantation ; et

c) la comparaison de la valeur en ligne de base avec le niveau d'expression d'ARNm détecté à l'étape b),

où une valeur en ligne de base inférieure à la valeur test dans le cas des gènes KRT15 prédit que le sujet ayant eu une transplantation rénale présente un risque accru de développer un RC.

2. Procédé selon la revendication 1, comprenant en outre :

a) la détection en tant que valeur en ligne de base du niveau d'expression d'ARNm correspondant aux gènes hoxB7, NPRL2, OS9, γ7 de protéine G, OBMCL, DFKZp558B1722, EST ID 32f4, hoxA7 et PRLR, lesdits gènes provenant d'une biopsie de tissu d'allogreffe spécifique d'un sujet de contrôle transplanté qui est connu pour ne pas développer de RC ;

b) la détection d'un niveau d'expression d'ARNm correspondant aux gènes identifiés en a) dans une biopsie de tissu d'allogreffe de rein obtenue dans les 4 à 7 mois suivant la transplantation chez un sujet test ayant eu une transplantation rénale ; et

c) la comparaison de la valeur en ligne de base avec le niveau d'expression d'ARNm détecté à l'étape b) ;

où une valeur en ligne de base inférieure à la valeur test dans le cas des gènes hoxB7, NPRL2, OS9, γ7 de protéine G, OBMCL, DFKZp558B1722 et EST ID 32f4, et supérieure à la valeur test dans le cas des gènes hoxA7 et PRLR, prédit que le sujet ayant eu une transplantation rénale présente un risque accru de développer un RC.

**3.** Procédé selon la revendication 1 ou 2, dans lequel la biopsie du tissu d'allogreffe est obtenue chez le sujet de contrôle le jour de la transplantation.

**4.** Procédé selon les revendications 1 à 3, dans lequel à l'étape b), le niveau d'expression d'ARNm correspondant au (x) gène(s) sélectionné(s) à l'étape a) dans une biopsie de tissu d'allogreffe de rein obtenue chez un sujet test ayant eu une transplantation rénale est détecté à environ 6 mois post-transplantation.

**5.** Procédé de surveillance du RC in vitro chez un sujet transplanté présentant un risque de développer un RC, comprenant a) l'obtention d'un échantillon pré-administration auprès d'un sujet ayant eu un rein transplanté avant l'administration d'un agent inhibiteur de RC, b) la détection du niveau d'expression d'ARNm correspondant au gène KRT15 dans l'échantillon pré-administration, c) l'obtention d'un ou de plusieurs échantillons post-administration du patient ayant eu un rein transplanté, d) la détection du niveau d'expression d'ARNm correspondant au gène KRT15 dans l'échantillon ou les échantillons de rein post-administration, e) la comparaison du niveau d'expression d'ARNm correspondant au gène KRT15 dans l'échantillon pré-administration avec le niveau d'expression d'ARNm dudit gène dans l'échantillon ou les échantillons post-administration, et f) l'ajustement de l'agent en conséquence.

**6.** Procédé selon la revendication 5, comprenant en outre

   a) la détection du niveau d'expression d'ARNm correspondant aux gènes hoxB7, NPRL2, OS9, γ7 de protéine G, OBMCL, DFKZp558B1722, EST ID 32f4, hoxA7 et PRLR, lesdits gènes provenant d'une biopsie de tissu d'allogreffe spécifique d'un sujet de contrôle transplanté qui est connu pour ne pas développer de RC ;
   b) la détection d'un niveau d'expression d'ARNm correspondant aux gènes identifiés en a) dans une biopsie de tissu d'allogreffe de rein obtenue dans les 4 à 7 mois suivant la transplantation chez un sujet test ayant eu une transplantation rénale ; et
   c) la comparaison du niveau de l'étape a) avec le niveau d'expression d'ARNm détecté à l'étape b),

où un niveau de l'étape a) inférieur au niveau de l'étape b) dans le cas des gènes hoxB7, NPRL2, OS9, γ7 de protéine G, OBMCL, DFKZp558B1722 et EST ID 32f4, et supérieur au niveau de l'étape b) dans le cas des gènes hoxA7 et PRLR, prédit que le sujet ayant eu une transplantation rénale présente un risque accru de développer un RC.

**7.** Procédé selon l'une quelconque des revendications 1 à 6, dans lequel le niveau d'expression d'ARNm d'un ou de plusieurs gènes est détecté par des techniques sélectionnées dans le groupe composé d'une analyse par transfert de Northern, d'une RT-PCR et d'une PCR quantitative en temps réel.

**8.** Utilisation du gène KRT15 en tant que biomarqueur pour le rejet chronique de greffes selon le procédé de la revendication 1.

**9.** Utilisation selon la revendication 8, comprenant en outre les gènes hoxB7, NPRL2, OS9, γ7 de protéine G, OBMCL, DFKZp558B1722, EST ID 32f4, hoxA7 et PRLR.

**10.** Utilisation selon la revendication 8 ou 9, en tant que biomarqueur pour le rejet chronique de greffes dans les 4 à 7 mois post-transplantation.

**11.** Utilisation selon l'une quelconque des revendications 8 à 10, en tant que biomarqueur d'un rejet chronique de greffes à environ 6 mois.

**EP 1 532 278 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0534858 A1